# EUROPEAN PATENT APPLICATION

(11) **EP 4 364 670 A1**
(43) Date of publication of application: **08.05.2024**
(21) Application number: 22207944.4
(22) Date of filing: 17.11.2022
(51) Int. Cl.: A61B 8/00

(54) **WEARABLE ULTRASOUND PATCHES**

(30) Priority: 07.11.2022 US 202263423208 P
(71) Applicant: Koninklijke Philips N.V., 5656 AG Eindhoven (NL)
(72) Inventor: HAARTSEN, Jaap Roger, Eindhoven (NL); HINTZEN, Annemijn, Eindhoven (NL); DEKKER, Ronald, 5656AG Eindhoven (NL)
(74) Representative: Philips Intellectual Property & Standards

(57) **Abstract**

The invention relates to wearable ultrasound patches. In order to place a patch (330), a method of placement and a patch (330) for placement are provided. The method of placing the patch comprises: connecting (Fig. 3b) a first ultrasound transducer (310) to the patch (330), finding a target position, separating (Fig. 3e) the first ultrasound transducer (310) from the patch (330), and connecting (Fig. 3f) a second ultrasound transducer (320) to the patch (330). This approach enables, amongst others, the use of a minimal functionality second ultrasound transducer (320), while accurately determining the target position with the first ultrasound transducer (310).

## Description

### FIELD OF THE INVENTION

The invention relates to a method for placing ultrasound patches and a wearable ultrasound patch.

### BACKGROUND OF THE INVENTION

Point-of-care ultrasound (POCUS) using handheld ultrasound devices such as the Philips Lumify is rapidly gaining popularity across a multitude of healthcare specialties. It is a portable, fast, real-time, non-invasive and safe (non-ionizing radiation) imaging technique performed by a physician at the bedside and is standard practice in obstetric, emergency and musculoskeletal medicine. However, POCUS also has some limitations. For example, it is operator dependent, both in terms of image interpretation and in terms of repeatability and reproducibility since probe placement is done manually. Also, since it is a handheld device, it is less suitable for applications where continuous or regular measurements are desired. In addition, because of the Covid-19 pandemic, the demand for solutions that require as little nurse-patient interaction as possible has risen. For this purpose, wearable ultrasound patches are emerging.

An ultrasound patch is an ultrasound transducer that is integrated within a patch, a wearable device that connects to the skin and monitors health parameters of a patient. Advantages of such an ultrasound patch is that it allows for continuous and repeatable measurements, and after placement, the caregiver workload and nurse-patient contact is limited. Ideally, these patches are small, wireless and low-cost. However, this is at the expense of functionality. For example, continuous high-resolution imaging requires a large transducer area, complex on-board electronics, high power consumption and a high data transfer rate. For monitoring purposes, a full image is not always needed. For example, for tracking a vein diameter, bladder diameter or muscle thickness, once the patch is placed at the right location, it is sufficient to scan along one or a couple of scanlines instead of scanning along many scanlines. Thus, patches used for monitoring may only have a minimum functionality and not always provide full ultrasound images. This enables the use of relatively cheap ultrasound patches, but comes at an increased difficulty of finding the correct place on the surface of a subject for these ultrasound patches.

### SUMMARY OF THE INVENTION

It is, inter alia, an object of the invention to provide a method for placement of ultrasound patches, and a patch that enables the placement method.

The invention is defined by the independent claims. Advantageous embodiments are defined in the dependent claims.

In an aspect of the invention, there is provided a method for placing a wearable ultrasound patch, the method comprising:
- connecting a first transducer array to a patch;
- moving the combination of the first transducer array and the patch over a surface of a subject to a target position;
- connecting the patch with the subject at the target position;
- separating the first transducer array from the patch; and
- connecting a second transducer array to the patch.

In this manner, a second ultrasound transducer array for monitoring purposes that does not produce ultrasound images suitable for placement can be placed without sacrificing on the target position accuracy. For example, a physician can choose the ultrasound transducer of their choice as the first ultrasound transducer (e.g., Philips Lumify), connect a patch to this transducer, find the target position, connect the patch to the subject, and then replace the first ultrasound transducer for a second ultrasound transducer. However, as described in more detail below, embodiments of the invention may provide additional and/or alternative advantages.

According to an embodiment, the step of connecting the first transducer array to the patch comprises:
- connecting the first transducer array to an adapter, and connecting the adapter to the patch; or
- connecting the adapter to the patch, and connecting the first transducer array to the adapter.

In some instances, the transducer of choice of the physician may not fit well or easily into the patch. In such cases, an adapter may be used to bridge size and/or shape differences between the patch and first transducer array.

According to an embodiment, the step of moving the combination of the first transducer array and the patch over a surface of a subject to a target position may further comprise:
- generating ultrasound images with the first ultrasound transducer array; and
- displaying the generated ultrasound images on a display.

In this manner the physician can visually, based on ultrasound images on a display, find the target position.

According to an embodiment, the first transducer array is part of an ultrasound probe configured to be held by a user. For example, any ultrasound transducer on the market such as any cart-based probes or the Philips Lumify probe.

According to an embodiment, the ultrasound probe is a hand-held ultrasound probe. For example, the Philips Lumify probe.

According to an embodiment, the second transducer array is part of an ultrasound probe configured to be used in a wearable ultrasound patch. For example, the second ultrasound transducer is part of a probe designed to be wearable on the surface of a subject. In other words, the design is small and minimalistic so as to occupy little space and not pose hindrances to the subject wearing the second transducer array patch combination. The combination of second transducer array and patch is commonly known as wearable ultrasound patch.

According to an embodiment, the method may further comprise:
- determining a first ultrasound signal parameter for generating an ultrasound image at the target location with the first ultrasound transducer array (310), and
- determining a second ultrasound signal parameter for generating an ultrasound image at the target location with the second ultrasound transducer array (310), wherein the second ultrasound signal parameter is based on the first ultrasound signal parameter.

In this manner, the ultrasound signal parameter (i.e., frequency, propagation speed, wavelength, amplitude, power and/or intensity etc.) used by the first transducer array may be conveyed to the second transducer array, such that the ultrasound signal parameter only needs to be set once. Thus, minimizing the computing and or manual labor to set the ultrasound signal parameters to monitor a particular feature within a subject from the target location. In an example, the ultrasound signal parameter used during the step of "moving the combination of the first transducer array and the patch over a surface of a subject to a target position" may be conveyed to the second transducer array.

According to some embodiments, the method further comprises:
- inserting a gel-pad into the patch, or
- connecting a first gel-pad to the first transducer array and connecting a second gel-pad to the second transducer array.

According to an aspect of the invention there is provided a patch for a wearable ultrasound transducer, the patch comprising:
- a first connection mechanism with a surface of a subject;
- a second connection mechanism with a first ultrasound transducer array; and
- a third connection mechanism with a second ultrasound transducer array.

The above patch thus enables the connection of a first ultrasound transducer array which may be contained in a typical ultrasound probe (e.g., Philips Lumify) and of a second transducer array that may be within a probe designed to be worn by a subject. In this manner, enabling the method of placing a wearable ultrasound patch as described above.

According to an embodiment, the second connection mechanism and the third connection mechanism are the same.

According to an embodiment, the first connection mechanism comprises any one of:
- an adhesive, wherein the adhesive connects the patch with the surface of the subject.
- a vacuum, wherein a vacuum is formed between the patch and the surface of the subject.

According to an embodiment, the second and/or third connection mechanisms comprise any one of:
- a click-based system configured to interlock the patch with the first ultrasound transducer and/or the second ultrasound transducer;
- a pressure-based system, wherein a force pushes the first ultrasound transducer and/or the second ultrasound onto the patch;
- an adhesion-based system, wherein an adhesive connects the patch with the first ultrasound transducer and/or the second ultrasound transducer; and
- a magnetic system, wherein a magnet connects the patch with the first ultrasound transducer and/or the second ultrasound transducer.

According to an embodiment, the second and/or third connection mechanisms further comprise:
- an adapter configured to bridge size and/or shape differences between the patch and the first ultrasound transducer and/or the second ultrasound transducer.

According to an embodiment, the patch may further comprise a cut-out or region of transparent or translucent material, through which the first transducer array and second transducer array can send acoustic waves into the subject and receive the reflections of the acoustic waves from the subject.

According to an embodiment, the patch may further comprise a gel pad holder configured to hold a gel pad, wherein the gel-pad is configured to be placed in the cut-out or region of transparent or translucent material:
- between the surface of the subject and the first transducer array and/or
- between the surface of the subject and the second transducer array.

These and other aspects of the invention will be apparent from and elucidated with reference to the embodiments described hereinafter.

### BRIEF DESCRIPTION OF THE DRAWINGS

Fig. 1 is an exemplary flow chart of a method according to an embodiment of the invention.
Fig. 2a-2e show exemplary locations for wearable ultrasound patch placements in lung ultrasound monitoring.
Fig. 3a-3f display graphically the method of Fig. 1 according to an embodiment of the present invention.
Fig. 4a shows a patch according to an embodiment of the invention.
Fig. 4b shows an adapter for use in an embodiment of the invention.
Fig. 4c shows a minimum functionality ultrasound probe for use in an embodiment of the invention.
Fig. 5 displays a typical ultrasound system for use in an embodiment of the invention.

### DETAILED DESCRIPTION OF EMBODIMENTS

The invention will be described herein with reference to the Figures. It should be understood that the description and specific examples provided, while indicating exemplary embodiments, are intended for purposes of illustration only, and not intended to limit the scope of the invention. It should also be understood that the figures are mere schematics and not drawn to scale. It should also be understood that the same reference numerals are used throughout the Figures to indicate the same or similar parts.

The invention provides a method and devices for placement of a wearable ultrasound patch.

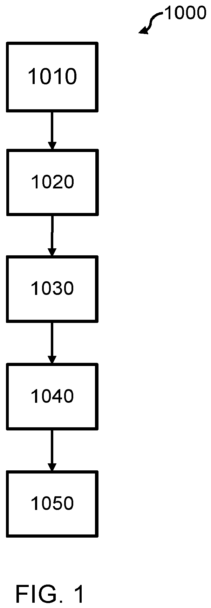

Fig. 1 displays a schematic of a method 1000 according to an embodiment of the present invention.

The method 1000 according to exemplary Fig. 1 describes the placement of a wearable ultrasound patch comprising the steps of:
i) Connecting 1010 a patch with a first transducer array. In this example, the patch may be a device suitable for placement on a surface of a subject. For example, an adhesive device and/or a vacuum device as described further below. The first transducer array may be a transducer array of an ultrasound probe such as the Philips Lumify. The connection between the patch and the first transducer array may further be enabled by any means, e.g.,
   - a click-based system configured to interlock the patch with the first ultrasound transducer and/or the second ultrasound transducer;
   - a pressure-based system, wherein a force pushes the first ultrasound transducer and/or the second ultrasound onto the patch;
   - an adhesion-based system, wherein an adhesive connects the patch with the first ultrasound transducer and/or the second ultrasound transducer; and
   - a magnetic system, wherein at least one magnet connects the patch with the first ultrasound transducer and/or the second ultrasound transducer.

In an optional step, the step 1010 of connecting the patch with the first transducer array may comprise:
- connecting the first transducer array to an adapter, and connecting the adapter to the patch; or
- connecting the adapter to the patch, and connecting the first transducer array to the adapter.

In this manner, the patch may be designed without or with limited prior knowledge of the form factor and/or design of the first transducer array. The adapter may thus enable a variety of first transducer arrays, for example 1D (one-dimensional), 1.XD, 2D and/or 3D transducer arrays, to be connected to the patch, and to be used according to the method 1000.

ii) Moving 1020 the combination of the first transducer array and the patch over a surface of a subject to a target position. In this step, a physician may thus scan into the body of a subject to determine a target position for monitoring of a particular feature of the subject as discussed in more detail below. In particular, the physician may visualize the ultrasound data in an ultrasound image on a display in order to determine a target position for a monitoring activity. In other examples, an algorithm may indicate the target position based on for example identified features in a feature detection assessment. In an example, the physician may adjust ultrasound signal parameters emitted from the first transducer including but not limited to: the acoustic wave frequency, propagation speed, wavelength, amplitude, power and/or intensity, to determine a target position for monitoring a particular feature or object within the subject. In an example, the ultrasound signal parameters emitted from the first transducer may be adjusted or set automatically (e.g., by a machine learning module in response to an imaging procedure or based on detected features).

iii) Connecting 1030 the patch to the surface of the subject at the target position. Once a suitable target position is determined, the patch is connected to the surface of the subject. This connection may be done via an adhesive similar to tape or plasters, for example, the physician may remove an adhesive protective layer below the patch and glue the patch onto the surface of the subject, or via any other means. For example, vacuum may be used to connect the patch to the surface of the subject.

iv) Separating or disconnecting 1040 the first transducer array from the patch. The first transducer array may be separated by pulling the first transducer array out, or by operating some mechanical, electronical or magnetic connection mechanism that releases the first transducer array from the patch and allows for its retrieval.

v) Connecting 1050 a second transducer array to the patch. The connection mechanism may be of any kind, for example, a click-based system, an adhesive, a magnetic system, etc.

Having determined a suitable target position, the first transducer array is disconnected from the patch and a second transducer array is inserted. In this manner, the first transducer array used for finding the target position is replaced by a second transducer array suitable for monitoring, and not necessarily including the functionality and/or capability of enabling a physician or an algorithm to determine the target location. It is obvious to a person skilled in the art that both transducer arrays may be suitable for determining the target position and for monitoring.

The first transducer array and second transducer array may comprise any transducer technology including but not limited to:
- a piezoelectric transducer array;
- a capacitive micromachined ultrasonic transducer array.

Connecting in the sense of steps 1010 and 1050 comprises enabling scanning of a subject by the first transducer array and the second transducer array through the patch. In some examples, the patch thus comprises a cut-out through which the first and second transducer arrays may send acoustic signals (soundwaves) into the body of the subject. In other examples the patch comprises a translucent or transparent region through which the acoustic signals may be transmitted.

In order to convey the acoustic energy (soundwaves) from the first transducer array and/or the second transducer array into the surface of the subject with minimal reflection and refraction, a connecting contact medium may be necessary. For this purpose, typically ultrasound gel is used. However, in wearable ultrasound patches, ultrasound gel may not be convenient since it may hinder the attachment of the patch onto the surface of the subject. As a result, gel-pads may be used that may be attached directly to the transducer array or inserted into the patch so as to be between the transducer array and the surface of the subject. By inserting a gel-pad into the patch, the ultrasound data quality may thus be improved. In some examples, the patch may thus further comprise a region and/or cut-out for a gel-pad. In accordance, the method 1000 may further comprise a step of connecting a gel-pad with the patch or the first and second transducer arrays. This optional step of placing the gel pad may be done at any stage, for example before connecting the first and/or second transducer array to the patch or thereafter.

The gel-pad according to the preceding paragraph may be a solid ultrasound gel-pad.

It is obvious to a person skilled in the art that the steps of method 1000 may be carried out in a different order as presented here. For example, the patch and the first ultrasound transducer may be connected during the examination. The physician may briefly remove the first transducer array from the surface of the subject to attach the patch and then re-place the first transducer array-patch combination on the surface of the subject.

According to method 1000, a physician (i.e., a sonographer, a radiologist or any other person operating an ultrasound transducer and/or placing an ultrasound patch) may thus chose an ultrasound transducer array of their liking to search for a target position on a surface of a subject (i.e., patient, person or any object that is being examined or scanned), wherein the target position allows for monitoring a particular feature the physician wishes to have monitored. In particular, the physician may use a conventional ultrasound probe which comprises the first ultrasound transducer array, and visualize ultrasound images on a display such as an ultrasound cart, phone, tablet or any other monitor to aid in the determination of the target position. Once a target position is found, the physician then connects the patch to the surface of the subject. In some examples, the physician may again verify the target position before removing the first transducer array. After removal of the first transducer array, a physician inserts a second transducer array. The second transducer array may be intended for monitoring purposes containing considerably fewer functionality than the first transducer array. In some examples, the second transducer array is a minimum functionality product, capable only of scanning along a few scanlines and incapable of generating complete ultrasound images. This second transducer array may however be capable of monitoring particular features of interest for an extended period of time, while not connected to any external device or power supply. In other words, the patch and second transducer array combination may be considered a wearable ultrasound patch. In other examples, the second transducer array may comprise similar or the same functionality as the first transducer array. In other examples the first transducer array and second transducer array may differ in size and/or shape.

In an example, the method may further comprise
- determining a first ultrasound signal parameter for generating an ultrasound image at the target location with the first ultrasound transducer array, and
- determining a second ultrasound signal parameter for generating an ultrasound image at the target location with the second ultrasound transducer array, wherein the second ultrasound signal parameter is based on the first ultrasound signal parameter.

In other words, the ultrasound signal parameters of the first transducer array (i.e., frequency, propagation speed, wavelength, amplitude, power and/or intensity etc.) may be conveyed to the second transducer array. For example, the optimal ultrasound signal parameters of the first transducer array to visualize a particular feature inside the subject from the target position may be conveyed to the second transducer array, so that the second transducer array can optimally monitor said feature from the target position. The step of conveying the ultrasound signal parameters may be carried out by a physician or may happen automatically, for example if both transducer arrays, the first and second transducer arrays, are connected to the same computing device. In an example, the ultrasound signal parameters of the first transducer array may be set by a physician. In an example, the ultrasound signal parameters of the first transducer array may be set automatically by machine learning or artificial intelligence. In an example, the physician may overwrite and/or correct the ultrasound signal parameters received by the second transducer array from the first transducer array.

In an example, the ultrasound signal parameters of the second transducer array may be set by a physician. In an example, the ultrasound signal parameters of the second transducer array may be set automatically by machine learning or artificial intelligence. In an example, the ultrasound signal parameters of the second transducer array may deviate by a given (i.e., pre-defined) amount from the ultrasound signal parameter of the first transducer array, for example to account for a different number of acoustic elements between the second and the first transducer arrays.

Information gathered by the second transducer array regarding the monitoring may be stored on local memory, or may be transmitted wired or wirelessly to an external memory. The transmission of the information may further take place during the monitoring examination or thereafter.

After the examination is complete, the second ultrasound transducer and patch combination may be disconnected from the surface of the subject.

In some examples, the patch is disposable.

In some examples, the first transducer array and/or the second transducer array are reusable.

In some examples the gel-pad is disposable.

The second transducer array in the meaning above may be used for any of the following applications:
- Diaphragmatic ultrasound monitoring, e.g., continuous atrophy detection, diaphragm dysfunction detection, weaning prediction and patient-ventilator asynchrony detection.
- Lung ultrasound monitoring, e.g., pleural effusion monitoring, atelectasis progression tracking, pneumonia progression tracking and lung sliding monitoring.
- Hemodynamic monitoring, e.g., blood flow and plaque monitoring.
- Fetal monitoring, e.g., fetal heart beat monitoring.
- Liver monitoring, e.g., monitoring for liver disease.
- Renal ultrasound, e.g., monitoring after transplantation, renal-blood flow monitoring
- Bladder monitoring, e.g., bladder volume, obstruction, foley catheter placement or malfunctions, bladder stones.
- Prostate enlargement monitoring
- Cardiovascular home monitoring, e.g., electrocardiograph monitoring, arterial pressure monitoring.
- Carotid artery narrowing monitoring, e.g., stroke monitoring, cardiac output monitoring, atrial fibrillation monitoring.
- Heart muscle function and cardiac output in an acute care setting, e.g., monitoring after a heart attack.
- Home ventilation monitoring to predict exacerbations.

Wearable ultrasound transducers may also be used for many other applications not mentioned here.

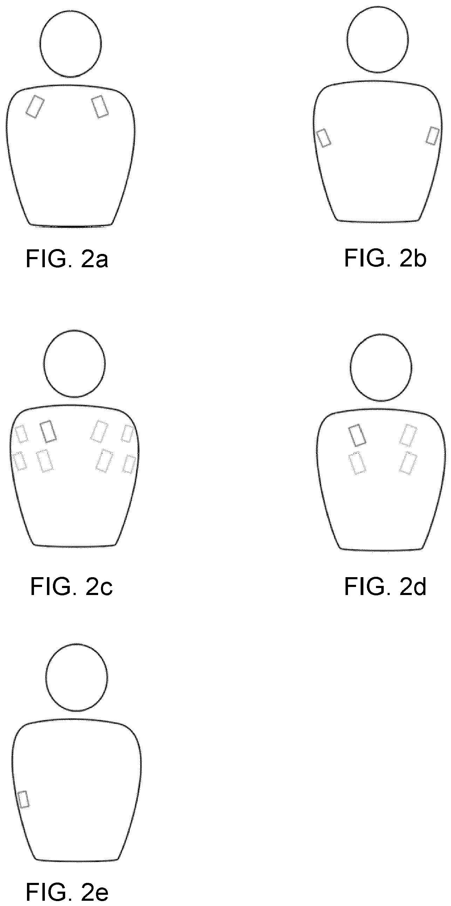

For illustrative purposes only, Fig. 2 displays exemplary placements of an ultrasound patch for various lung monitoring examinations:
- Fig. 2a shows placements for examining and/or monitoring lung sliding (upper anterior zones);
- Fig. 2b shows placements for examining and/or monitoring pleural effusion (lower lateral zones);
- Fig. 2c shows placements for examining and/or monitoring pneumonia (risk zones or all zones);
- Fig. 2d shows placements for examining and/or monitoring atelectasis (risk zones, only anterior); and
- Fig. 2e shows placements for examining and/or monitoring diaphragm monitoring (lateral right, at base of diaphragm).

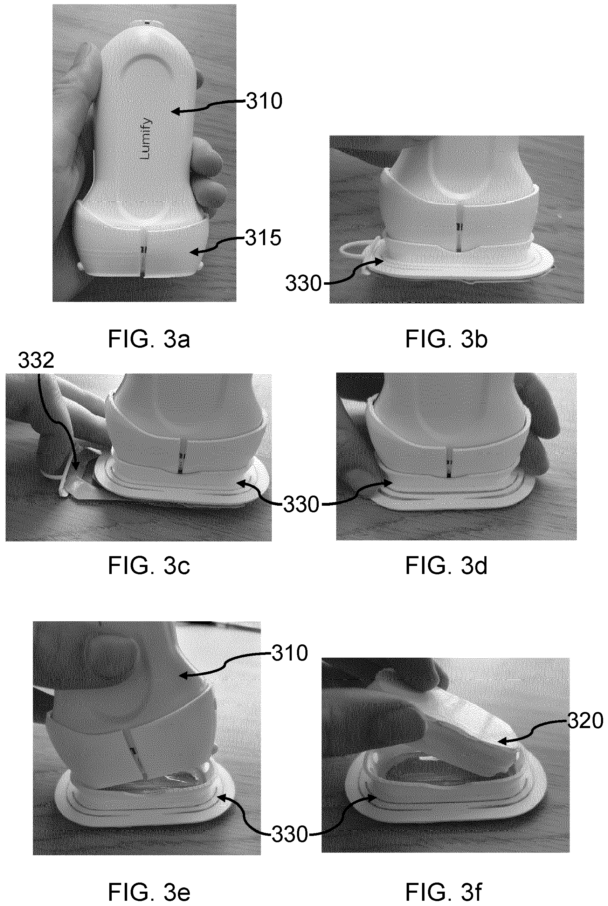

Figs. 3a to 3f display an example of the method 1000 as executed in practice.

Fig. 3a displays a Philips Lumify ultrasound probe 310, wherein the first transducer array is part of the ultrasound probe 310, connected to an adapter 315.

Fig. 3b displays the ultrasound probe 310 connected to the patch 330.

Fig. 3c shows how a liner 332 covering an adhesive layer below the patch 330 may be removed.

Fig. 3d shows how the patch may be connected with the surface of a subject by pushing down the adhesive layer onto the surface of the subject. In particular, the patch 330 has a spring like construction to enable movement of the adhesive layer towards the surface of the subject.

Fig. 3e displays the separation or removal of the ultrasound probe 310 from the patch 330.

Fig. 3f shows the connection of the second transducer array 320 with the patch 330, here as part of a minimum functionality ultrasound probe, such that the combination of the patch and the second transducer array is smaller in size and better suited as a wearable ultrasound patch. At the same time, the minimum functionality design enables increased up-time (i.e., the time of operation when not connected to a power supply) and reduces the overall financial cost of manufacturing.

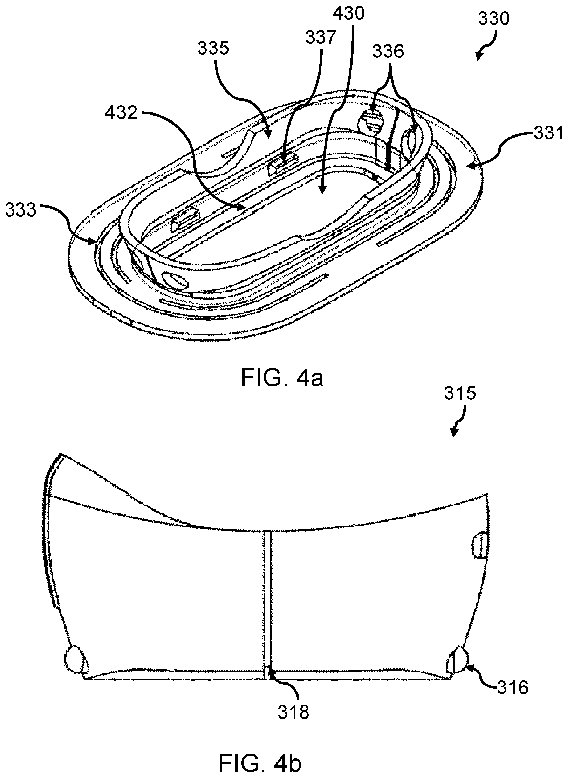

Fig. 4a shows a patch 330 for use in a wearable ultrasound patch according to an embodiment of the present invention. In particular, Fig. 4a shows a patch with:
- a first connection mechanism to connect the patch with a surface of a subject;
- a second connection mechanism to connect the patch to a first ultrasound transducer; and
- a third connection mechanism to connect the patch to a second ultrasound transducer.

The first connection mechanism comprises a plate 331, wherein a first side (the lower side in Fig. 4a) is configured to be placed on the surface of a subject. The first side of the plate 331 may further comprise an adhesive layer enabling the connection between the patch and the surface of the subject. The adhesive layer may further be covered by a removable liner 332 (Fig. 3c displays the liner 332 being removed). The liner 332, is for example, configured to stop the first side of plate 331 from adhering to the surface of the subject while moving the first transducer array-patch combination according to step 1020 of method 1000. Once a physician wishes to connect the patch to the surface of a subject, the physician may remove the liner as shown in Fig. 3c thus connecting the patch to the surface of the subject. The plate 331 of the first connection mechanism further comprises various cut-outs 333 enabling a spring like behavior of the plate 331. This spring-like behavior is advantageous to compensate for skin movements of the subject that is being monitored. For example, tension and/or compression of the skin while the subject is moving. In other words, the cut-outs 333 enable a more secure and comfortable adhesion with the subject.

It is noted that also other first connection mechanisms are envisaged by the present invention such as vacuum options wherein a vacuum is generated between the patch and the surface of the subject to hold the patch at the target position. For example, a method to generate such a vacuum is to integrate one or multiple suction cups at the bottom of plate 331. Such a suction cup can have multiple form factors such as circular or it can have the form of plate 331 such that one suction cup covers the entire bottom surface of the plate. Additionally, a mechanism may be provided to generate vacuum in the suction cup. Such a mechanism may include a mechanism to increase the inner volume of the suction cup or to remove the air from the suction cup, e.g., by a piston or a miniature pump.

The second and third connection mechanisms shown in Fig. 4a are the same. However, this is not necessary and using different second and third connection mechanisms is entirely possible and envisaged by the present invention. The second connection mechanism in Fig. 4a (thus also the third connection mechanism) is configured to connect a transducer array to the patch. In some embodiments, an adapter is connected to the second connection mechanism, wherein the adapter connects to a transducer array. The connection mechanism comprises a cone-shaped opening 335 with cut-outs 336 and protrusions 337 to enable a secure connection (i.e., interlocking) between the transducer array or an adapter and the patch.

The patch 330 may further comprise a cut-out or region of transparent or translucent material 430. This cut-out or region may be designed to hold a gel-pad. For example, a gel-pad may be inserted through the cone-shaped opening 335 and placed in the opening 430 while the edges of the gel-pad may be resting on surface 432. The gel-pad may be any gel-pad, but typically a solid gel-pad made out of mostly aqueous materials. In an example, the backside of the gel-pad, the side that is in contact with the first and/or second transducer array may be slightly cone shaped where the highest point is in the center such that when connecting either of the first and second transducer arrays the air is pressed outwards. As a result, no air bubbles should remain at the interface between the gel-pad and the transducer surface.

Fig. 4b shows an adapter 315 to connect an ultrasound probe with the patch 330 of Fig. 4a according to an embodiment of the present invention.

In particular, the adapter 315 may be designed to bridge size and/or shape differences between an ultrasound transducer array 310 and the patch 330. In this particular embodiment, the adapter 315 is designed to fit the transducer array 310 of the Philips Lumify hand-held ultrasound probe. However, other forms for the adapter 315, depending on the form and form factor of the first transducer array are also envisaged by the present invention.

The adapter 315 may further comprise protrusions 316 designed to fit into the cut-outs 336 of the patch enabling an interlocking between the patch and the adapter. By extension thus enabling an interlocking between the patch and the ultrasound probe connected to the adapter.

The adapter 315 may further comprise a cut-out 318. This cut-out 318 may be used to increase flexibility of the adapter and reduce manufacturing tolerances, or may also be used to work together with the second connection mechanism by enabling pushing the protrusions 316 towards each other, thus making it easier to take the protrusions 316 out of the cut-outs 336 of the patch.

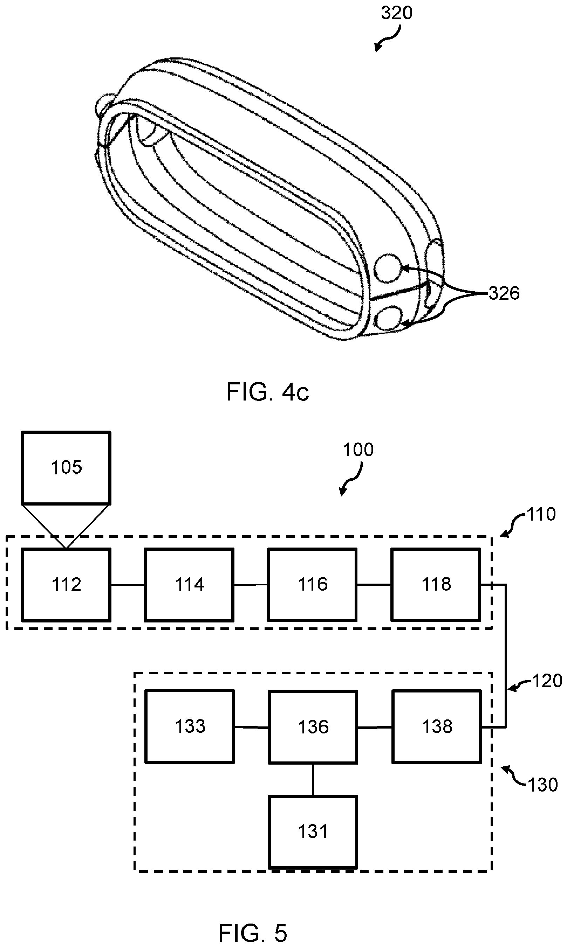

Fig. 4c shows an ultrasound probe 320 designed to form, when in combination with the patch 330 of Fig. 4a, a wearable ultrasound patch.

The ultrasound probe 320, similarly as the adapter 315, contains protrusions 326, designed to fit into the cut-outs 336 of the patch 330.

The ultrasound probe 320 may further comprise the second transducer array, wherein the second transducer array is configured for monitoring a particular feature of a subject as described above. Other forms for the ultrasound probe 320, different to the one depicted in Fig. 4c, wherein the form depends on the form and form factor of the second transducer array are also envisaged by the present invention. In an example, the second transducer array may be connected to the patch 330 via an adapter similar to adapter 315.

The patch 330, the adapter 315 and the casing of the ultrasound probe 320 may be of any suitable material (e.g., metal, plastic, particularly polyethylene, combination of materials, etc.) and manufacturing method (e.g., 3D printed, casted, assembled from different parts, etc.).

While a specific adapter and second and third connection mechanisms have been described above, namely that of a clicking system, other alternatives are also envisaged by the present invention. For example, the first and second transducer array may be connected to the patch 330 by the use of an adhesive. In an example, the first and second transducer array may be connected to the patch via a pressure system. In particular, a physician may exert pressure on the first and/or second transducer array while pushed against the surface of the subject, thus keeping the patch and the transducer array together. In yet other examples, a magnetic system may be used wherein in magnets are integrated into the patch and the first and/or second transducer array or the adapter 315, wherein the magnets are configured to keep the patch and the transducer array connected via a magnetic force.

An exemplary ultrasound system according to the present disclosure is disclosed in Fig. 5. Fig. 5 shows a schematic diagram of an ultrasound imaging system 100. The system 100 includes an ultrasound imaging probe 110 in communication with a host 130 over a communication interface or link 120. The probe 110 may include a transducer array 112, a beamformer 114, a processor 116, and a communication interface 118. The transducer array 112 may be the first transducer array and/or the second transducer array. The host 130 may include a display 131, a processor 136, a communication interface 138, and a memory 133. The host 130 and/or the processor 136 of the host 130 may also be in communication with other types of systems or devices in replacement or addition to the here mentioned systems such as an external memory, external display, a subject tracking system, an inertial measurement unit, etc. It is understood that the beamformer may also be a microbeamformer. It is further understood that the components as shown here may also be configured in alternate arrangements. For example, the processor 116 and/or the beamformer 114 may be located outside of the probe 110 and/or the display 131 and/or memory 133 may be located outside of the host 130.

In some embodiments, the probe 110 is an ultrasound imaging device including a housing configured for handheld operation by a user. The transducer array 112 can be configured to obtain ultrasound data while the user grasps the housing of the probe 110 such that the transducer array 112 is positioned adjacent to or in contact with a patient's skin. The probe 110 is configured to obtain ultrasound data of anatomy within the patient's body while the probe 110 is positioned outside of the patient's body. In some embodiments, the probe 110 can be a patch-based external ultrasound probe. For example, the probe may be a hemodynamic patch.

The transducer array 112 emits ultrasound signals towards an anatomical object 105 of a patient and receives echo signals reflected from the object 105 back to the transducer array 112. The transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x-dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a patient's anatomy. In some embodiments, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The beamformer 114 is coupled to the transducer array 112. The beamformer 114 controls the transducer array 112, for example, for transmission of the ultrasound signals and reception of the ultrasound echo signals. In some embodiments, the beamformer 114 may apply a time-delay to signals sent to individual acoustic transducers within an array in the transducer array 112 such that an acoustic signal is steered in any suitable direction propagating away from the probe 110. The beamformer 114 may further provide image signals to the processor 116 based on the response of the received ultrasound echo signals. The beamformer 114 may include multiple stages of beamforming. The beamforming can reduce the number of signal lines for coupling to the processor 116. In some embodiments, the transducer array 112 in combination with the beamformer 114 may be referred to as an ultrasound imaging component. The beamformer 114 may also be a microbeamformer.

The processor 116 is coupled to the beamformer 114. The processor 116 may also be described as a processor circuit, which can include other components in communication with the processor 116, such as a memory, beamformer 114, communication interface 118, and/or other suitable components. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 138 may receive the image signals. The communication interface 138 may be substantially similar to the communication interface 118. The host 130 may be any suitable computing and display device, such as a workstation, a personal computer (PC), a laptop, a tablet, or a mobile phone.

The processor 136 is coupled to the communication interface 138. The processor 136 may also be described as a processor circuit, which can include other components in communication with the processor 136, such as the memory 133, the communication interface 138, and/or other suitable components. The processor 136 can be configured to generate image data from the image signals received from the probe 110. The processor 136 can apply advanced signal processing and/or image processing techniques to the image signals. An example of image processing includes conducting a pixel level analysis to evaluate whether there is a change in the color of a pixel, which may correspond to an edge of an object (e.g., the edge of an anatomical feature). In some embodiments, the processor 136 can form a three-dimensional (3D) volume image from the image data. In some embodiments, the processor 136 can perform real-time processing on the image data to provide a streaming video of ultrasound images of the object 105.

The memory 133 is coupled to the processor 136. The memory 133 can be configured to store patient information, measurements, data, or files relating to a patient's medical history, history of procedures performed, anatomical or biological features, characteristics, or medical conditions associated with a patient, computer readable instructions, such as code, software, or other application, as well as any other suitable information or data. The memory 133 may be located within the host 130. There may also be an additional external memory, or an external memory in replacement of memory 133. An external memory may be a cloud-based server or an external storage device, located outside of the host 130 and in communication with the host 130 and/or processor 136 of the host via a suitable communication link as disclosed with reference to communication link 120. Patient information may include measurements, data, files, other forms of medical history, such as but not limited to ultrasound images, ultrasound videos, and/or any imaging information relating to the patient's anatomy. The patient information may include parameters related to an imaging procedure such a probe position and/or orientation.

The display 131 is coupled to the processor 136. The display 131 may be a monitor or any suitable display or display device. The display 131 is configured to display the ultrasound images, image videos, and/or any imaging information of the object 105.

The system 100 may be used to assist a sonographer or operator in performing an ultrasound scan. The scan may be performed in a point-of-care setting. In some instances, the host 130 is a console or movable cart. In some instances, the host 130 may be a mobile device, such as a tablet, a mobile phone, or portable computer. In yet other examples the host is a server on a cloud and an external display connects to the host in the cloud. During an imaging procedure, the ultrasound system 100 can acquire an ultrasound image of a region of interest of a subject.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A method (1000) for placing a patch (330), the method comprising:
- connecting (1010) a first transducer array (310) to the patch (330);
- moving (1020) the combination of the first transducer array (310) and the patch (330) over a surface of a subject to a target position;
- connecting (1030) the patch (330) with the subject at the target position;
- separating (1040) the first transducer array (310) from the patch (330); and
- connecting (1050) a second transducer array (320) to the patch (330).

2. The method of claim 1, wherein the step of connecting the first transducer array to the patch comprises:
- connecting the first transducer array (310) to an adapter (315), and connecting the adapter (315) to the patch (320); or
- connecting the adapter (315) to the patch (330), and connecting the first transducer array (310) to the adapter (315).

3. The method of any of the preceding claims, wherein the step of moving the combination of the first transducer array and the patch over a surface of a subject to a final position further comprises,
- generating ultrasound images with the first ultrasound transducer array (310); and
- displaying the generated ultrasound images on a display (131).

4. The method of any of the preceding claims, wherein the first transducer array (310) is part of an ultrasound probe configured to be held by a user.

5. The method of claim 4, wherein the ultrasound probe is a hand-held ultrasound probe.

6. The method of any of the preceding claims, wherein the second transducer array (320) is part of a wearable ultrasound probe configured to be used in the patch (330).

7. The method of any of the preceding claims, further comprising:
- determining a first ultrasound signal parameter for generating an ultrasound image at the target location with the first ultrasound transducer array (310), and
- determining a second ultrasound signal parameter for generating an ultrasound image at the target location with the second ultrasound transducer array (310), wherein the second ultrasound signal parameter is based on the first ultrasound signal parameter.

8. The method of any of the preceding claims, further comprising
- inserting a gel-pad into the patch (330), or
- connecting a first gel-pad to the first transducer array (310), and connecting a second gel-pad to the second transducer array (320).

9. A patch (330) for a wearable ultrasound transducer, the patch comprising:
- a first connection mechanism with a surface of a subject;
- a second connection mechanism with a first ultrasound transducer array; and
- a third connection mechanism with a second ultrasound transducer array.

10. The patch of claim 9, wherein the second connection mechanism and the third connection mechanism are the same.

11. The patch of claim 9 or 10, wherein the first connection mechanism comprises any one of:
- an adhesive, wherein the adhesive connects the patch with the surface of the subject;
- a vacuum, wherein a vacuum is formed between the patch and the surface of the subject.

12. The patch of any of claims 9 to 11, wherein the second and/or third connection mechanisms comprise any one of:
- a click-based system configured to interlock the patch with the first ultrasound transducer and/or the second ultrasound transducer;
- a pressure-based system, wherein a force pushes the first ultrasound transducer and/or the second ultrasound onto the patch;
- an adhesion-based system, wherein an adhesive connects the patch with the first ultrasound transducer and/or the second ultrasound transducer; and
- a magnetic system, wherein a magnet connects the patch with the first ultrasound transducer and/or the second ultrasound transducer.

13. The patch of any of claims 9 to 12, wherein the second and/or third connection mechanisms further comprise:
- an adapter (315) configured to bridge size and/or shape differences between the patch (330) and the first ultrasound transducer (310) and/or the second ultrasound transducer (320).

14. The patch of any of claims 9 to 13, wherein the patch (330) further comprises a cut-out or region of transparent or translucent material (430), through which the first transducer array (310) and the second transducer array (320) can send acoustic waves into the subject and receive reflections of the acoustic waves from the subject.

15. The patch of claim 14, further comprising a gel pad holder configured to hold a gel pad, wherein the gel-pad is configured to be placed in the cut-out or region of transparent or translucent material (430):
- between the surface of the subject and the first transducer array (310) and/or
- between the surface of the subject and the second transducer array (320).
